# EUROPEAN PATENT APPLICATION

(11) **EP 4 768 015 A1**
(43) Date of publication of application: **01.07.2026**
(21) Application number: 24855822.3
(22) Date of filing: 20.08.2024
(51) Int. Cl.: A61K 8/64, A61K 8/37, A61K 8/34, A61K 8/06, A61Q 19/00

(54) **EMULSION AND PREPARATION METHOD THEREFOR**

(30) Priority: 21.08.2023 CN 202311056263
(71) Applicant: Bloomage Biotechnology Corporation Limited, Jinan, Shandong 250101 (CN)
(72) Inventor: SUN, Xin, Beijing 102629 (CN); QU, Wenjie, Beijing 102629 (CN); WANG, Ruiyan, Beijing 102629 (CN); ZOU, Songyan, Beijing 102629 (CN); LUO, Xiping, Beijing 102629 (CN); CHANG, Kuan, Beijing 102629 (CN); LIN, Bochuan, Beijing 102629 (CN)
(74) Representative: Huang, Liwei
(86) International application number: PCT/CN2024/113470
(87) International publication number: WO 2025/040100

(57) **Abstract**

The present invention belongs to the technical field of daily chemical industry, and specifically relates to an emulsion and a preparation method thereof. The emulsion provided by the present invention comprises an emulsifier, glycerol, an oil-phase active substance, pentaerythrityl tetra(ethylhexanoate) and water; wherein the emulsifier is a lipopeptide; the weight percentage of the lipopeptide relative to glycerol is not less than 3%; the weight ratio of the oil-phase active substance to pentaerythrityl tetra(ethylhexanoate) is 1:(3 to 13); the ratio of the total weight of the lipopeptide and glycerol to the total weight of the oil-phase active substance and pentaerythrityl tetra(ethylhexanoate) is 1:(3 to 10); and the ratio of the total weight of the lipopeptide, glycerol, oil-phase active substance and pentaerythrityl tetra(ethylhexanoate) to the weight of water is 1:(1 to 4). By compounding sodium surfactin with glycerol and pentaerythrityl tetra(ethylhexanoate), the emulsion can effectively inhibit the diffusion of the oil-phase active substance at an emulsion interface, reduce the oxidative degradation of the oil-phase active substance, and greatly prolong the effective action time of the oil-phase active substance in the emulsion.

## Description

The present invention claims the priority of the Chinese patent application filed with the Chinese Patent Office on August 21, 2023, with an application number of 202311056263.X and entitled "Emulsion and Preparation Method Thereof", the contents of which are incorporated herein by reference in their entireties.

### TECHNICAL FIELD

The present invention belongs to the technical field of daily chemical industry, and specifically relates to an emulsion and a preparation method thereof.

### BACKGROUND

For a long time, the stabilization of oil-phase active substances has been a research hotspot in the development of cosmetics. Long-chain conjugated molecules represented by retinol, phenolic molecules represented by equol, polyhydric alcohol molecules represented by ascorbyl palmitate, and other such substances are highly prone to degradation, discoloration and deterioration due to light and heat in daily environments, posing great challenges to their application in cosmetics. At present, the stabilization strategies for oil-phase active substance in cosmetics mainly focus on obtaining encapsulants such as liposomes, aqueous gels, microemulsions and emulsions based on the self-assembly of various surfactants; therefore, the consumption of oil-phase active substance by oxidants is inhibited through a dense surfactant interfacial film, and an effect of stabilizing the active substance is further achieved.

As secondary metabolites (usually mixtures) produced during microbial fermentation, lipopeptides are a type of biosurfactants composed of β-amino or β-hydroxy fatty acids (lipophilic groups) and peptide chains or peptide rings (hydrophilic groups). Sodium surfactin, a lipopeptide-based biosurfactant also known as surfactin, is produced by fermentation of *Bacillus subtilis* strains. The hydrophilic group of its molecule consists of a cyclic peptide; and the cyclic peptide composed of seven amino acids possesses abundant hydrogen bond acceptors and donors, making it a bio-based anionic surfactant. Lipopeptides have advantages such as environmental friendliness and safety, and have thus become a research hotspot in the field of surfactants at present. Currently, lipopeptides have been applied to the preparation of emulsions and gels in the field of daily chemical industry. However, in the prior art, there are relatively few relevant researches on the application of lipopeptides to protect oil-phase active substance from excessive degradation over time. In addition, the protective effect of lipopeptides on oil-phase active substance is also associated with other formulated components, and the influencing factors are relatively complex. Therefore, it is necessary to provide a product that exerts excellent antioxidant protective effect on oil-phase active substance by using lipopeptides, and simultaneously address the problems of complicated preparation processes and poor universality commonly existing in various encapsulants in the prior art.

### SUMMARY

To address the above problems, an emulsion and a preparation method thereof are provided. The emulsion provided in the present invention is prepared by compounding sodium surfactin with glycerol and pentaerythrityl tetra(ethylhexanoate). In the prepared emulsion containing oil-phase active substance, the addition of sodium surfactin can effectively inhibit the diffusion of oil-phase active substance at an emulsion interface, thereby reducing the oxidative degradation of oil-phase active substance and prolonging the effective action time of oil-phase active substance in the emulsion. In addition, the preparation process is simple, which solves the problems of complicated preparation process and poor universality that are commonly associated with addressing the problems of easy degradation of oil-phase active substance in the prior art.

According to one aspect of the present invention, an emulsion is provided. The emulsion includes an emulsifier, glycerol, an oil-phase active substance, pentaerythrityl tetra(ethylhexanoate) and water;
the emulsifier is lipopeptide;
the weight percentage of the lipopeptide relative to glycerol is not less than 3%;
the weight ratio of the oil-phase active substance to pentaerythrityl tetra(ethylhexanoate) is 1:(3 to 13);
the ratio of the total weight of the lipopeptide and glycerol to the total weight of the oil-phase active substance and pentaerythrityl tetra(ethylhexanoate) is 1:(3 to 10);
the ratio of the total weight of the lipopeptide, glycerol, oil-phase active substance and pentaerythrityl tetra(ethylhexanoate) to the weight of water is 1:(1 to 4).

Further, the weight percentage of the lipopeptide relative to glycerol is selected from 3%, 4%, 5%, 6%, 7%, 8%, 9%, 10%, 11%, 12%, 13%, 14%, 15%, 16%, 17%, 18%, 19%, 20% and any percentage therebetween.

Further, the weight ratio of the oil-phase active substance to pentaerythrityl tetra(ethylhexanoate) is selected from 1:4, 1:5, 1:6, 1:7, 1:8, 1:9, 1:10, 1:11, 1:12 and any value therebetween.

Further, the ratio of the total weight of the lipopeptide and glycerol to the total weight of the oil-phase active substance and pentaerythrityl tetra(ethylhexanoate) is selected from 1:4, 1:5, 1:6, 1:7, 1:8, 1:9 and any value therebetween.

Further, the ratio of the total weight of the lipopeptide, glycerol, oil-phase active substance and pentaerythrityl tetra(ethylhexanoate) to the weight of water is selected from 1:1.5, 1:2, 1:2.5, 1:3, 1:3.5 and any value therebetween.

The emulsion provided in the present invention, compounded with the above components in a specific ratio, can form a microemulsion gel and thus form an oil-in-water emulsion during the dilution process. This emulsion has the performance advantages of small particle size and high stability. Owing to the large hydrophilic head groups of lipopeptide, hydrogen bonds can form among the cyclic peptides to hinder the diffusion of oxidative species from the aqueous phase to the oil phase, thereby achieving the effect of protecting and stabilizing the active substance in the oil phase and inhibiting their oxidative deterioration and degradation.

Optionally, the weight percentage of the lipopeptide relative to glycerol is not less than 5%, and further optionally 5% to 12%; and/or,
the weight ratio of the oil-phase active substance to the pentaerythrityl tetra(ethylhexanoate) is 1:5 to 11.

Optionally, the lipopeptide include any one or more selected from sodium surfactin, iturin, fengycin, daptomycin, polymyxin, bacillomycin L, plipastatin and bacillomycin D.

Optionally, the oil-phase active substance includes one or more selected from retinol, retinyl acetate, retinyl propionate, retinyl palmitate, bakuchiol, equol, nervonic acid, ascorbyl palmitate, ascorbyl tetrapalmitate and phenylethyl resorcinol.

According to another aspect of the present invention, a preparation method for any of the above emulsions is provided, and the method includes the following steps:
1) dissolving the lipopeptide in glycerol, and heating and stirring the mixture to obtain a solution A;
2) dissolving the oil-phase active substance in pentaerythrityl tetra(ethylhexanoate), and stirring the mixture to obtain a solution B; and
3) adding the solution A to the solution B under the conditions of constant temperature water bath and stirring to obtain the oil gel; and
4) adding the aqueous phase to the oil gel under stirring conditions to allow thorough mixing, to obtain the lipopeptide-containing emulsion.

Optionally, the lipopeptide in step 1) include any one or more selected from sodium surfactin, iturin, fengycin, daptomycin, polymyxin, bacillomycin L, plipastatin and bacillomycin D.

Optionally, the temperature for heating and stirring in step 1) is 75 to 85°C.

Optionally, the temperature of the constant temperature water bath in step 3) is 45 to 55°C.

Optionally, the stirring speed in step 3) is not less than 600 rpm.

Optionally, the stirring temperature in step 4) is 20 to 30°C. After the formation of the oil gel, solutions A and B have been mixed relatively uniformly during the stirring and dilution process in step 4), so only mild heating or room temperature conditions are required at this time, and room temperature can be selected since it is more conducive to the preservation of the oil-phase active substance.

Optionally, in step 3), solution B is added to solution A slowly in batches; further optionally, after the solution B added each time is fully emulsified, more solution B is added subsequently.

According to the last aspect of the present invention, applications of any of the above emulsions in cosmetics are provided.

The beneficial effects of the present invention include but are not limited to:
1. The emulsion of the present invention has the advantages of small particle size and high stability, and no demulsification occurs even after the emulsion undergoes high-temperature storage, thermal cycling, shear tests and other tests.
2. The emulsion of the present invention can effectively protect oil-phase active substance from oxidative degradation, inhibit the degradation and discoloration of oil-phase active ingredients such as retinyl propionate, and significantly improve the retention rate of oil-phase active substance in the emulsion over time.
3. The emulsion of the present invention is characterized by simple preparation process and low equipment requirements, resulting in low production cost and easy operation. The preparation of the emulsion does not require the use of complex equipment such as high-pressure homogenizers, and an emulsion with excellent antioxidant protection for oil-phase active substance can be prepared only by simple stirring and mixing.
4. The emulsion of the present invention features simple components, mild raw materials and low irritation, which conforms to the trend of environmentally friendly and safe raw materials in the daily chemical industry.

### BRIEF DESCRIPTION OF THE DRAWINGS

The accompanying drawings described herein are provided for a further understanding of the present invention and constitute a part of the present invention, and the schematic examples of the present invention and their descriptions are intended to illustrate the present invention and shall not constitute an undue limitation on the present invention. In the accompanying drawings:
Fig. 1 is a schematic diagram of the molecular structure of sodium surfactin;
Fig. 2 is a schematic diagram showing the photographs of the oil phase gels of Examples 1 to 3 of the present invention (reference numerals 1, 2 and 3 in the figure correspond to Examples 1, 2 and 3, respectively);
Fig. 3 is a schematic diagram showing the photographs of the emulsions of Examples 1 to 3 of the present invention (reference numerals 1 and 2 in the figure correspond to Examples 1 and 2, respectively);
Fig. 4 is a schematic diagram of the stability test results of Example 1 and Comparative Example 2 of the present invention (reference numerals 1 and D2 in the figure correspond to Example 1 and Comparative Example 2, respectively);
Fig. 5 is a schematic diagram of the stability test results of Example 2 and Comparative Example 4 of the present invention (reference numerals 2 and D4 in the figure correspond to Example 2 and Comparative Example 4, respectively);
Fig. 6 is a schematic diagram showing the photographs of Comparative Example 1 and Comparative Example 3 of the present invention (reference numerals D1 and D3 in the figure correspond to Comparative Example 1 and Comparative Example 3, respectively);
Fig. 7 is a schematic diagram showing the photograph of Comparative Example 7 of the present invention (reference numeral D7 in the figure corresponds to Comparative Example 7);
Fig. 8 is a schematic diagram of the stability test results of Example 9 of the present invention (reference numeral 9 in the figure corresponds to Example 9);
Fig. 9 is a schematic diagram of the stability test results of Comparative Example 9 of the present invention (reference numeral D9 in the figure corresponds to Comparative Example 9).

### DETAILED DESCRIPTION OF THE EMBODIMENTS

The present invention is described in detail below with reference to examples, but the present invention is not limited to these examples. Unless otherwise specified, all the raw materials and catalysts used in the examples of the present invention are purchased through commercial channels.

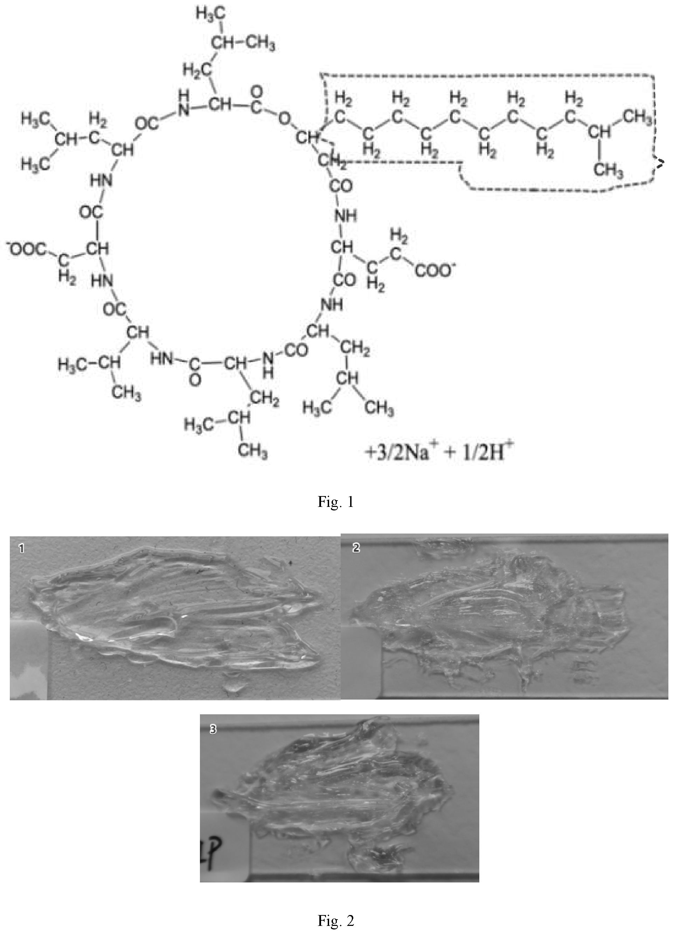

As shown in Fig. 1 which is a schematic diagram of the molecular structure of sodium surfactin, there have been relevant reports in the prior art on the applications of sodium surfactin as an emulsifier to the preparation of daily necessities such as gels and emulsions. In the prior art, sodium surfactin is mainly added to act as an emulsifier, enabling a mixed liquid of two or more immiscible components to form a stable emulsion. In the present invention, in the research of the inventors on the application of sodium surfactin to emulsion preparation, it was found that in the emulsion containing oil-phase active substances prepared by the combined use of sodium surfactin with glycerol and pentaerythrityl tetra(ethylhexanoate), the addition of sodium surfactin can effectively inhibit the diffusion of oil-phase active substances at the emulsion interface, thereby reducing the oxidative degradation of oil-phase active substances and prolonging the effective action time of oil-phase active substances in the emulsion.

During further experimental studies, the inventors found that the protective effect of sodium surfactin in inhibiting the oxidation of oil-phase active substances is closely related to the selection of components compounded with sodium surfactin and the addition ratios of the corresponding components. For example, the use of oleic acid, caprylic/capric triglyceride(GTCC) and other substances commonly employed in emulsion preparation cannot achieve a favorable synergistic effect, and may even fail to form an emulsion, thereby limiting their compounded use with sodium surfactin.

In the present invention, based on the application of sodium surfactin in improving the retention rate of oil-phase active substance over time, the inventors investigated the effects of components suitable for compounding with sodium surfactin and their addition ratios, and obtained an emulsion solution that can significantly improve the retention rate of oil-phase active substance over time, thereby greatly enhancing the preservation effect of oil-phase active substance. The solution of the present invention is further illustrated below through specific examples, comparative examples and test examples.

### Example 1

An emulsion includes phase A, phase B and phase C, wherein phase A includes the following raw materials in parts by weight: 1 g of sodium surfactin and 20 g of glycerol; phase B includes the following raw materials in parts by weight: 15 g of retinyl propionate and 75 g of pentaerythrityl tetra(ethylhexanoate); and phase C is 160 g of water.

The emulsion is prepared by the following method:
dissolving 1 g of sodium surfactin in 20 g of glycerol, and heating and stirring at 80°C for 20 min to obtain a clear and stable phase D (A); dissolving 15 g of retinyl propionate in 75 g of the liquid oil phase pentaerythrityl tetra(ethylhexanoate) to obtain an oil phase (B) containing active substance;
maintaining phase D (A) and the oil phase (B) in a water bath at 50°C;
under a constant temperature water bath at 50°C, slowly adding the oil phase (B) while stirring phase D (A) at 600 rpm, ensuring that the oil phase added each time is fully emulsified and dissolved before adding more oil phase, to obtain an oil gel containing the active substance, wherein as the oil phase content increases, the system viscosity increases gradually, and an oil gel containing the active substance is finally obtained;
adding 160 g of the aqueous phase (C) while stirring the oil gel at 500 rpm, and stirring for 10 min until the oil gel is completely dissolved, to obtain an oil-in-water (o/w) emulsion containing retinyl propionate.

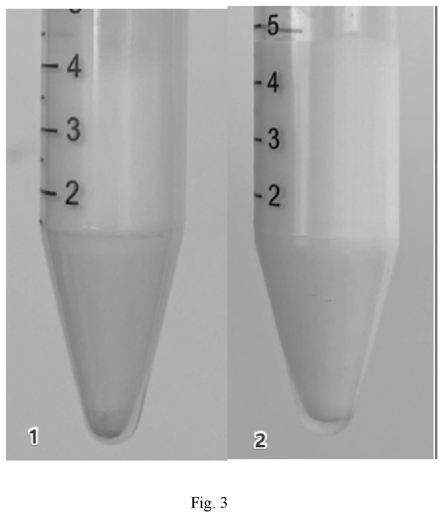

The photograph of the oil phase gel prepared in Example 1 is shown in the image labeled 1 in Fig. 2, and the photograph of the emulsion is shown in the image labeled 1 in Fig. 3.

### Example 2

An emulsion includes phase A, phase B and phase C, wherein phase A includes the following raw materials in parts by weight: 1 g of sodium surfactin and 20 g of glycerol; phase B includes the following raw materials in parts by weight: 15 g of bakuchiol and 75 g of pentaerythrityl tetra(ethylhexanoate); and phase C is 160 g of water.

The emulsion is prepared by the following method:
dissolving 1 g of sodium surfactin in 20 g of glycerol, and heating and stirring at 80°C for 20 min to obtain a clear and stable phase D (A); dissolving 15 g of bakuchiol in 75 g of the liquid oil phase pentaerythrityl tetra(ethylhexanoate) to obtain an oil phase (B) containing active substance;
maintaining phase D (A) and the oil phase (B) in a water bath at 50°C;
under a constant temperature water bath at 50°C, slowly adding the oil phase (B) while stirring phase D (A) at 600 rpm, ensuring that the oil phase added each time is fully emulsified and dissolved before adding more oil phase, to obtain an oil gel containing the active substance, wherein as the oil phase content increases, the system viscosity increases gradually, and an oil gel containing the active substance is finally obtained;
adding 160 g of the aqueous phase (C) while stirring the oil gel at 500 rpm, and stirring for 10 min until the oil gel is completely dissolved, to obtain an oil-in-water (o/w) emulsion containing bakuchiol.

The photograph of the oil phase gel prepared in Example 2 is shown in the image labeled 2 in Fig. 2, and the photograph of the emulsion is shown in the image labeled 2 in Fig. 3.

### Example 3

An emulsion includes phase A, phase B and phase C, wherein phase A includes the following raw materials in parts by weight: 1 g of sodium surfactin and 20 g of glycerol; phase B includes the following raw materials in parts by weight: 15 g of ascorbyl tetrapalmitate and 75 g of pentaerythrityl tetra(ethylhexanoate); and phase C is 160 g of water.

The emulsion is prepared by the following method:
dissolving 1 g of sodium surfactin in 20 g of glycerol, and heating and stirring at 80°C for 20 min to obtain a clear and stable phase D (A); dissolving 15 g of ascorbyl tetrapalmitate in 75 g of the liquid oil phase pentaerythrityl tetra(ethylhexanoate) to obtain an oil phase (B) containing active substance;
maintaining phase D (A) and the oil phase (B) in a water bath at 50°C;
under a constant temperature water bath at 50°C, slowly adding the oil phase (B) while stirring phase D (A) at 600 rpm, ensuring that the oil phase added each time is fully emulsified and dissolved before adding more oil phase, to obtain an oil gel containing the active substance, wherein as the oil phase content increases, the system viscosity increases gradually, and an oil gel containing the active substance is finally obtained;
adding 160 g of the aqueous phase (C) while stirring the oil gel at 500 rpm, and stirring for 10 min until the oil gel is completely dissolved, to obtain an oil-in-water (o/w) emulsion containing ascorbyl tetrapalmitate.

The photograph of the oil gel prepared in Example 3 is shown in the image labeled 3 in Fig. 2.

### Example 4

An emulsion includes phase A, phase B and phase C, wherein phase A includes the following raw materials in parts by weight: 1 g of sodium surfactin and 20 g of glycerol; phase B includes the following raw materials in parts by weight: 15 g of retinyl propionate and 75 g of pentaerythrityl tetra(ethylhexanoate); and phase C is 111 g of water.

The emulsion is prepared by the following method:
dissolving 1 g of sodium surfactin in 20 g of glycerol, and heating and stirring at 80°C for 20 min to obtain a clear and stable phase D (A); dissolving 15 g of retinyl propionate in 75 g of the liquid oil phase pentaerythrityl tetra(ethylhexanoate) to obtain an oil phase (B) containing active substance;
maintaining phase D (A) and the oil phase (B) in a water bath at 50°C;
under a constant temperature water bath at 50°C, slowly adding the oil phase (B) while stirring phase D (A) at 600 rpm, ensuring that the oil phase added each time is fully emulsified and dissolved before adding more oil phase, to obtain an oil gel containing the active substance, wherein as the oil phase content increases, the system viscosity increases gradually, and an oil gel containing the active substance is finally obtained;
adding 111 g of the aqueous phase (C) while stirring the oil gel at 500 rpm, and stirring for 10 min until the oil gel is completely dissolved, to obtain an oil-in-water (o/w) emulsion containing retinyl propionate.

### Example 5

An emulsion includes phase A, phase B and phase C, wherein phase A includes the following raw materials in parts by weight: 1 g of sodium surfactin and 20 g of glycerol; phase B includes the following raw materials in parts by weight: 15 g of retinyl propionate and 75 g of pentaerythrityl tetra(ethylhexanoate); and phase C is 444 g of water.

The emulsion is prepared by the following method:
dissolving 1 g of sodium surfactin in 20 g of glycerol, and heating and stirring at 80°C for 20 min to obtain a clear and stable phase D (A); dissolving 15 g of retinyl propionate in 75 g of the liquid oil phase pentaerythrityl tetra(ethylhexanoate) to obtain an oil phase (B) containing active substance;
maintaining phase D (A) and the oil phase (B) in a water bath at 50°C;
under a constant temperature water bath at 50°C, slowly adding the oil phase (B) while stirring phase D (A) at 600 rpm, ensuring that the oil phase added each time is fully emulsified and dissolved before adding more oil phase, to obtain an oil gel containing the active substance, wherein as the oil phase content increases, the system viscosity increases gradually, and an oil gel containing the active substance is finally obtained;
adding 444 g of the aqueous phase (C) while stirring the oil gel at 500 rpm, and stirring for 10 min until the oil gel is completely dissolved, to obtain an oil-in-water (o/w) emulsion containing retinyl propionate.

### Example 6

An emulsion includes phase A, phase B and phase C, wherein phase A includes the following raw materials in parts by weight: 1 g of sodium surfactin and 20 g of glycerol; phase B includes the following raw materials in parts by weight: 10 g of retinyl propionate and 80 g of pentaerythrityl tetra(ethylhexanoate); and phase C is 160 g of water.

The emulsion is prepared by the following method:
dissolving 1 g of sodium surfactin in 20 g of glycerol, and heating and stirring at 80°C for 20 min to obtain a clear and stable phase D (A); dissolving 10 g of retinyl propionate in 80 g of the liquid oil phase pentaerythrityl tetra(ethylhexanoate) to obtain an oil phase (B) containing active substance;
maintaining phase D (A) and the oil phase (B) in a water bath at 50°C;
under a constant temperature water bath at 50°C, slowly adding the oil phase (B) while stirring phase D (A) at 600 rpm, ensuring that the oil phase added each time is fully emulsified and dissolved before adding more oil phase, to obtain an oil gel containing the active substance, wherein as the oil phase content increases, the system viscosity increases gradually, and an oil gel containing the active substance is finally obtained;
adding 160 g of the aqueous phase (C) while stirring the oil gel at 500 rpm, and stirring for 10 min until the oil gel is completely dissolved, to obtain an oil-in-water (o/w) emulsion containing retinyl propionate.

### Example 7

An emulsion includes phase A, phase B and phase C, wherein phase A includes the following raw materials in parts by weight: 0.45 g of sodium surfactin and 8.55 g of glycerol; phase B includes the following raw materials in parts by weight: 15 g of retinyl propionate and 75 g of pentaerythrityl tetra(ethylhexanoate); and phase C is 160 g of water.

The emulsion is prepared by the following method:
dissolving 0.45 g of sodium surfactin in 8.55 g of glycerol, and heating and stirring at 80°C for 20 min to obtain a clear and stable phase D (A); dissolving 15 g of retinyl propionate in 75 g of the liquid oil phase pentaerythrityl tetra(ethylhexanoate) to obtain an oil phase (B) containing active substance;
maintaining phase D (A) and the oil phase (B) in a water bath at 50°C;
under a constant temperature water bath at 50°C, slowly adding the oil phase (B) while stirring phase D (A) at 600 rpm, ensuring that the oil phase added each time is fully emulsified and dissolved before adding more oil phase, to obtain an oil gel containing the active substance, wherein as the oil phase content increases, the system viscosity increases gradually, and an oil gel containing the active substance is finally obtained;
adding 160 g of the aqueous phase (C) while stirring the oil gel at 500 rpm, and stirring for 10 min until the oil gel is completely dissolved, to obtain an oil-in-water (o/w) emulsion containing retinyl propionate.

### Example 8

An emulsion includes phase A, phase B and phase C, wherein phase A includes the following raw materials in parts by weight: 1.5 g of sodium surfactin and 28.5 g of glycerol; phase B includes the following raw materials in parts by weight: 15 g of retinyl propionate and 75 g of pentaerythrityl tetra(ethylhexanoate); and phase C is 200 g of water.

The emulsion is prepared by the following method:
dissolving 1.5 g of sodium surfactin in 28.5 g of glycerol, and heating and stirring at 80°C for 20 min to obtain a clear and stable phase D (A); dissolving 15 g of retinyl propionate in 75 g of the liquid oil phase pentaerythrityl tetra(ethylhexanoate) to obtain an oil phase (B) containing active substance;
maintaining phase D (A) and the oil phase (B) in a water bath at 50°C;
under a constant temperature water bath at 50°C, slowly adding the oil phase (B) while stirring phase D (A) at 600 rpm, ensuring that the oil phase added each time is fully emulsified and dissolved before adding more oil phase, to obtain an oil gel containing the active substance, wherein as the oil phase content increases, the system viscosity increases gradually, and an oil gel containing the active substance is finally obtained;
adding 200 g of the aqueous phase (C) while stirring the oil gel at 500 rpm, and stirring for 10 min until the oil gel is completely dissolved, to obtain an oil-in-water (o/w) emulsion containing retinyl propionate.

### Example 9

An emulsion includes phase A, phase B and phase C, wherein phase A includes the following raw materials in parts by weight: 0.75 g of sodium surfactin and 6.75 g of glycerol; phase B includes the following raw materials in parts by weight: 2 g of phenylethyl resorcinol and 20.5 g of pentaerythrityl tetra(ethylhexanoate); and phase C is 70 g of water.

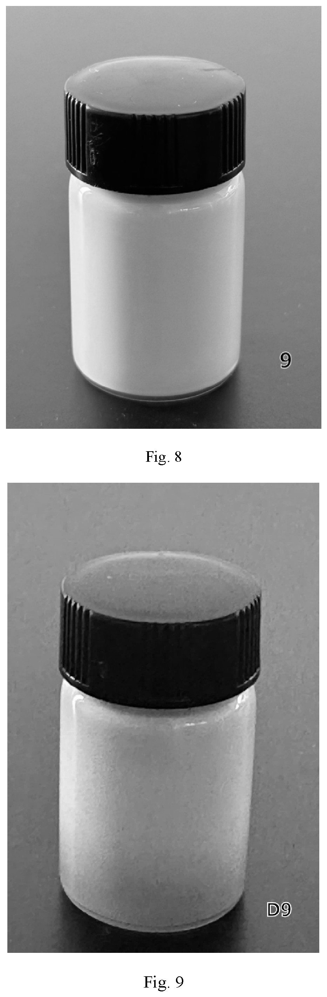

The emulsion is prepared by the following method:
dissolving 0.75 g of sodium surfactin in 6.75 g of glycerol, and heating and stirring at 80°C for 20 min to obtain a clear and stable phase D (A); dissolving 2 g of phenylethyl resorcinol in 20.5 g of the liquid oil phase pentaerythrityl tetra(ethylhexanoate) to obtain an oil phase (B) containing active substance;
maintaining phase D (A) and the oil phase (B) in a water bath at 50°C;
under a constant temperature water bath at 50°C, slowly adding the oil phase (B) while stirring phase D (A) at 600 rpm, ensuring that the oil phase added each time is fully emulsified and dissolved before adding more oil phase, to obtain an oil gel containing the active substance, wherein as the oil phase content increases, the system viscosity increases gradually, and an oil gel containing the active substance is finally obtained;
adding 70 g of the aqueous phase (C) while stirring the oil gel at 500 rpm, and stirring for 10 min until the oil gel is completely dissolved, to obtain an oil-in-water (o/w) emulsion containing phenylethyl resorcinol. The stability test results are shown in the image labeled 9 in Fig. 8.

### Comparative Example 1

An emulsion includes phase A, phase B and phase C, wherein phase A includes the following raw materials in parts by weight: 1 g of sodium surfactin and 20 g of glycerol; phase B includes the following raw materials in parts by weight: 15 g of retinyl propionate and 75 g of oleic acid; and phase C is 160 g of water.

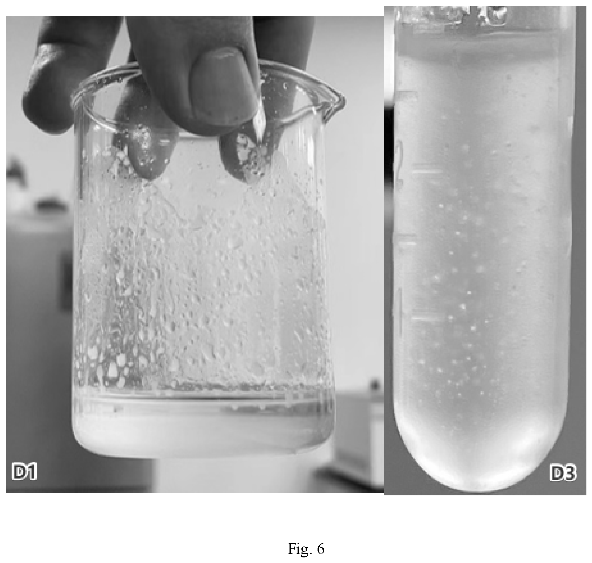

The emulsion is prepared by the following method:
dissolving 1 g of sodium surfactin in 20 g of glycerol, and heating and stirring at 80°C for 20 min to obtain a clear and stable phase D (A); dissolving 15 g of retinyl propionate in 75 g of the liquid oil phase oleic acid to obtain an oil phase (B) containing active substance;
maintaining phase D (A) and the oil phase (B) in a water bath at 50°C;
under a constant temperature water bath at 50°C, slowly adding the oil phase (B) while stirring phase D (A) at 600 rpm, wherein it is found that the two phases cannot be miscibilized or emulsified. The result is shown in the image labeled D1 in Fig. 6.

### Comparative Example 2

An emulsion includes phase A, phase B and phase C, wherein phase A includes the following raw materials in parts by weight: 1 g of sodium surfactin and 20 g of glycerol; phase B includes the following raw materials in parts by weight: 15 g of retinyl propionate and 75 g of GTCC; and phase C is 160 g of water.

The emulsion is prepared by the following method:
dissolving 1 g of sodium surfactin in 20 g of glycerol, and heating and stirring at 80°C for 20 min to obtain a clear and stable phase D (A); dissolving 15 g of retinyl propionate in 75 g of the liquid oil phase GTCC to obtain an oil phase (B) containing active substance;
maintaining phase D (A) and the oil phase (B) in a water bath at 50°C;
under a constant temperature water bath at 50°C, slowly adding the oil phase (B) while stirring phase D (A) at 600 rpm, ensuring that the oil phase added each time is fully emulsified and dissolved before adding more oil phase, to obtain an oil gel containing the active substance, wherein as the oil phase content increases, the system viscosity increases gradually, and an oil gel containing the active substance is finally obtained;
adding 160 g of the aqueous phase (C) while stirring the oil gel at 500 rpm, and stirring for 10 min until the oil gel is completely dissolved, to obtain an oil-in-water (o/w) emulsion containing retinyl propionate.

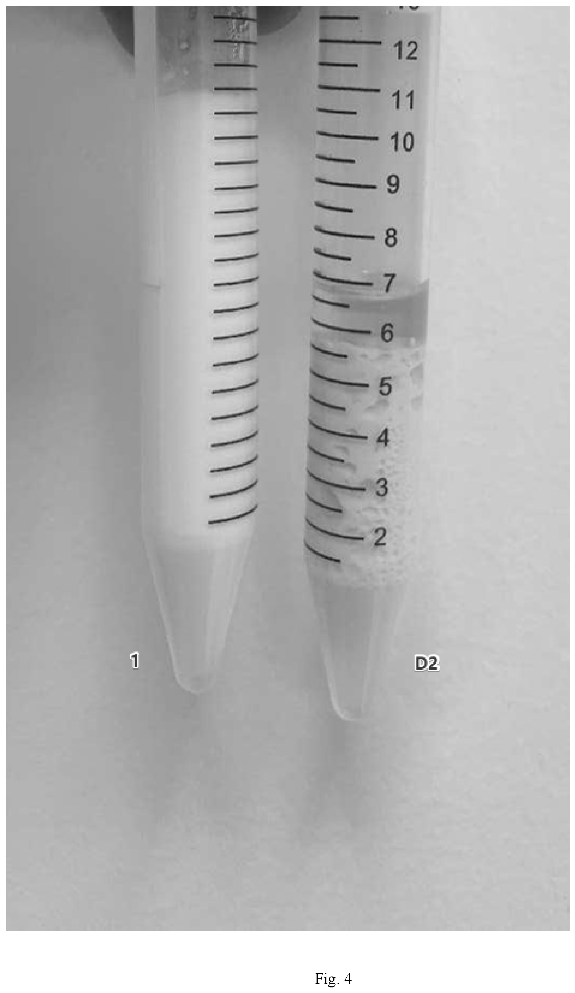

The result is shown in the image labeled D2 in Fig. 4. The result indicates that the emulsion has poor storage stability and leads to precipitation, discoloration and other problems.

### Comparative Example 3

An emulsion includes phase A, phase B and phase C, wherein phase A includes the following raw materials in parts by weight: 1 g of sodium surfactin and 20 g of butylene glycol; phase B includes the following raw materials in parts by weight: 15 g of retinyl propionate and 75 g of pentaerythrityl tetra(ethylhexanoate); and phase C is 160 g of water.

The emulsion is prepared by the following method:
dissolving 1 g of sodium surfactin in 20 g of butylene glycol, and heating and stirring at 80°C for 20 min while failing to obtain a clear and stable phase D (A); dissolving 15 g of bakuchiol in 75 g of the liquid oil phase pentaerythrityl tetra(ethylhexanoate) to obtain an oil phase (B) containing active substance;
maintaining phase D (A) and the oil phase (B) in a water bath at 50°C;
under a constant temperature water bath at 50°C, slowly adding the oil phase (B) while stirring phase D (A) at 600 rpm, wherein an oil phase gel cannot be formed, and the result is shown in the image labeled D3 in Fig. 6.

### Comparative Example 4

An emulsion includes phase A, phase B and phase C, wherein phase A includes the following raw materials in parts by weight: 1 g of sodium surfactin and 20 g of glycerol; phase B includes the following raw materials in parts by weight: 15 g of bakuchiol and 75 g of GTCC; and phase C is 160 g of water.

The emulsion is prepared by the following method:
dissolving 1 g of sodium surfactin in 20 g of glycerol, and heating and stirring at 80°C for 20 min to obtain a clear and stable phase D (A); dissolving 15 g of bakuchiol in 75 g of the liquid GTCC to obtain an oil phase (B) containing active substance;
maintaining phase D (A) and the oil phase (B) in a water bath at 50°C;
under a constant temperature water bath at 50°C, slowly adding the oil phase (B) while stirring phase D (A) at 600 rpm, ensuring that the oil phase added each time is fully emulsified and dissolved before adding more oil phase, to obtain an oil gel containing the active substance, wherein as the oil phase content increases, the system viscosity increases gradually, and an oil gel containing the active substance is finally obtained;
adding 160 g of the aqueous phase (C) while stirring the oil gel at 500 rpm, and stirring for 10 min until the oil gel is completely dissolved, to obtain an oil-in-water (o/w) emulsion containing bakuchiol.

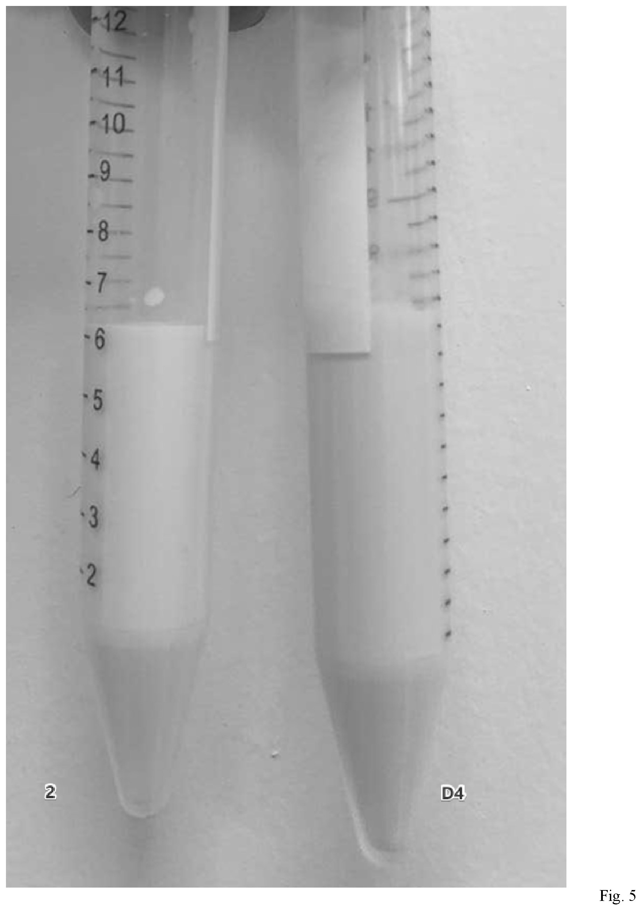

The result is shown in the image labeled D4 in Fig. 5. The result indicates that the emulsion has poor storage stability and leads to precipitation, discoloration and other problems.

### Comparative Example 5

An emulsion includes phase A, phase B and phase C, wherein phase A includes the following raw materials in parts by weight: 1 g of sodium surfactin and 20 g of glycerol; phase B includes the following raw materials in parts by weight: 15 g of retinyl propionate and 75 g of pentaerythrityl tetra(ethylhexanoate); and phase C is 100 g of water.

The emulsion is prepared by the following method:
dissolving 1 g of sodium surfactin in 20 g of glycerol, and heating and stirring at 80°C for 20 min to obtain a clear and stable phase D (A); dissolving 15 g of retinyl propionate in 75 g of the liquid oil phase pentaerythrityl tetra(ethylhexanoate) to obtain an oil phase (B) containing active substance;
maintaining phase D (A) and the oil phase (B) in a water bath at 50°C;
under a constant temperature water bath at 50°C, slowly adding the oil phase (B) while stirring phase D (A) at 600 rpm, ensuring that the oil phase added each time is fully emulsified and dissolved before adding more oil phase, to obtain an oil gel containing the active substance, wherein as the oil phase content increases, the system viscosity increases gradually, and an oil gel containing the active substance is finally obtained;
adding 100 g of the aqueous phase (C) while stirring the oil gel at 500 rpm and stirring for 10 min, wherein the oil gel cannot be completely dissolved, and obtaining a highly viscous and opaque mixture which does not fall within the category of emulsions.

### Comparative Example 6

An emulsion includes phase A, phase B and phase C, wherein phase A includes the following raw materials in parts by weight: 0.5 g of sodium surfactin and 20.5 g of glycerol; phase B includes the following raw materials in parts by weight: 15 g of retinyl propionate and 75 g of pentaerythrityl tetra(ethylhexanoate); and phase C is 160 g of water.

The emulsion is prepared by the following method:
dissolving 0.5 g of sodium surfactin in 20.5 g of glycerol, and heating and stirring at 80°C for 20 min to obtain a clear and stable phase D (A); dissolving 15 g of retinyl propionate in 75 g of the liquid oil phase pentaerythrityl tetra(ethylhexanoate) to obtain an oil phase (B) containing active substance;
maintaining phase D (A) and the oil phase (B) in a water bath at 50°C;
under a constant temperature water bath at 50°C, slowly adding the oil phase (B) while stirring phase D (A) at 600 rpm, wherein it is found that the oil phase cannot be completely emulsified or dissolved in phase D, and free oil phase always exists. This indicates that the ratio of lipopeptide to glycerol is too low to form an oil gel, thereby failing to form an emulsion.

### Comparative Example 7

An emulsion includes phase A, phase B and phase C, wherein phase A includes the following raw materials in parts by weight: 0.4 g of sodium surfactin and 8 g of glycerol; phase B includes the following raw materials in parts by weight: 15 g of retinyl propionate and 75 g of pentaerythrityl tetra(ethylhexanoate); and phase C is 138 g of water.

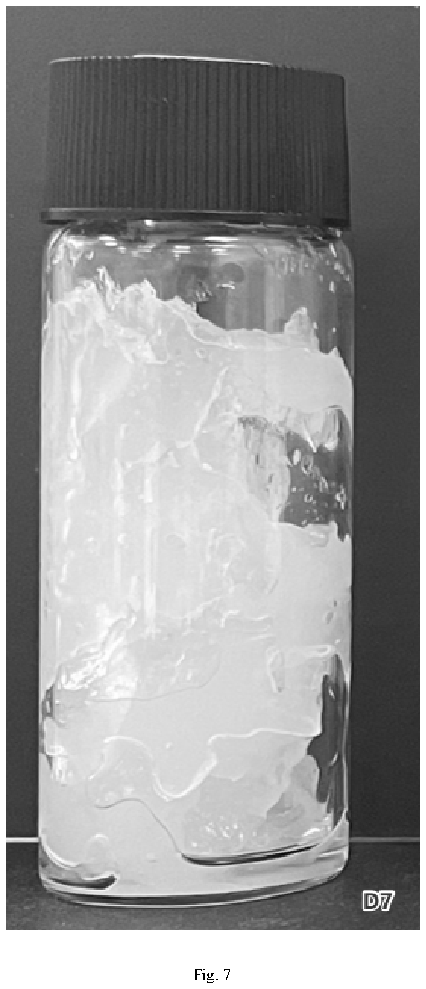

The emulsion is prepared by the following method:
dissolving 0.4 g of sodium surfactin in 8 g of glycerol, and heating and stirring at 80°C for 20 min to obtain a clear and stable phase D (A); dissolving 15 g of retinyl propionate in 75 g of the liquid oil phase pentaerythrityl tetra(ethylhexanoate) to obtain an oil phase (B) containing active substance;
maintaining phase D (A) and the oil phase (B) in a water bath at 50°C;
under a constant temperature water bath at 50°C, slowly adding the oil phase (B) while stirring phase D (A) at 600 rpm, wherein it is found that the oil phase cannot be completely emulsified or dissolved in phase D, and free oil phase always exists. This indicates that the ratio of phase A to phase B is too low to form an oil phase gel, thereby failing to form an emulsion. The result is shown in the image labeled D7 in Fig. 7.

### Comparative Example 8

An emulsion includes phase A, phase B and phase C, wherein phase A includes the following raw materials in parts by weight: 1 g of sodium surfactin and 20 g of glycerol; phase B includes the following raw materials in parts by weight: 30 g of retinyl propionate and 60 g of pentaerythrityl tetra(ethylhexanoate); and phase C is 160 g of water.

The emulsion is prepared by the following method:
dissolving 1 g of sodium surfactin in 20 g of glycerol, and heating and stirring at 80°C for 20 min to obtain a clear and stable phase D (A); dissolving 30 g of retinyl propionate in 60 g of the liquid oil phase pentaerythrityl tetra(ethylhexanoate) to obtain an oil phase (B) containing active substance;
maintaining phase D (A) and the oil phase (B) in a water bath at 50°C;
under a constant temperature water bath at 50°C, slowly adding the oil phase (B) while stirring phase D (A) at 600 rpm, ensuring that the oil phase added each time is fully emulsified and dissolved before adding more oil phase, to obtain an oil gel containing the active substance, wherein as the oil phase content increases, the system viscosity increases gradually, and an oil gel containing the active substance is finally obtained;
adding 160 g of the aqueous phase (C) while stirring the oil gel at 500 rpm, and stirring for 10 min until the oil gel is completely dissolved, to obtain an oil-in-water (o/w) emulsion containing retinyl propionate.

### Comparative Example 9

An emulsion includes phase A, phase B and phase C, wherein phase A includes the following raw materials in parts by weight: 0.4 g of sodium surfactin and 7.1 g of glycerol; phase B includes the following raw materials in parts by weight: 2 g of phenylethyl resorcinol and 28 g of pentaerythrityl tetra(ethylhexanoate); and phase C is 62.5 g of water.

The emulsion is prepared by the following method:
dissolving 0.4 g of sodium surfactin in 7.1 g of glycerol, and heating and stirring at 80°C for 20 min to obtain a clear and stable phase D (A); dissolving 2 g of phenylethyl resorcinol in 28 g of the liquid oil phase pentaerythrityl tetra(ethylhexanoate) to obtain an oil phase (B) containing active substance;
maintaining phase D (A) and the oil phase (B) in a water bath at 50°C;
under a constant temperature water bath at 50°C, slowly adding the oil phase (B) while stirring phase D (A) at 600 rpm, ensuring that the oil phase added each time is fully emulsified and dissolved before adding more oil phase, to obtain an oil gel containing the active substance, wherein as the oil phase content increases, the system viscosity increases gradually, and an oil gel containing the active substance is finally obtained;
adding 62.5 g of the aqueous phase (C) while stirring the oil gel at 500 rpm, and stirring for 10 min until the oil gel is completely dissolved, to obtain an oil-in-water (o/w) emulsion containing phenylethyl resorcinol, wherein the stability test results are shown in the image labeled D9 in Fig. 9.

### Test Example 1

Experimental method for determination of active substance degradation rate: The degradation rate of active substances such as retinyl propionate, bakuchiol, and phenylethyl resorcinol was determined by a high-performance liquid chromatography-ultraviolet (HPLC-UV) detector. Specifically, the prepared emulsions of Examples 1 to 9, Comparative Examples 2, 4 and 8 were placed in a constant temperature incubator at 50°C. At 7 d, 15 d and 28 d intervals, the emulsions were dissolved in methanol, and the degradation rates were determined via high-performance liquid chromatography. The specific conditions of the high-performance liquid chromatography were as follows: a C18 reversed-phase chromatographic column (250*4.6 mm, 5 µm) was adopted; the mobile phase consisted of 98 vol% methanol and 2 vol% water; the flow rate of the mobile phase was 1 mL/min; the detection wavelength was 325 nm; and quantitative detection was performed via the external standard method.

Table 1 shows the component proportions of the emulsions of Examples 1 to 9 and Comparative Examples 1 to 9, and Table 2 shows the results of the determination experiment of active substance degradation rate in Test Example 1.

**Table 1: Component Proportions of Emulsions**

| | Phase D (A) | | | B | | | B:A | C | C: (A+B) |
|---|---|---|---|---|---|---|---|---|---|
| | Lipope ptide (g) | Glycerol (g) | Lipopeptid e: glycerol | Oil phase (g) | Active substance (g) | Oil phase: active substance | | Aque ous phas e (g) | |
| Example 1 | 1 | 20 | 5% | 75 g pentaerythrit yl tetra(ethylhe xanoate) | 15 g retinyl propionat e | 5:1 | 4.3:1 | 160 | 1.4:1 |
| Example 2 | 1 | 20 | 5% | 75 g pentaerythrit yl tetra(ethylhe xanoate) | 15 g bakuchiol | 5:1 | 4.3:1 | 160 | 1.4:1 |
| Example 3 | 1 | 20 | 5% | 75 g pentaerythrit yl tetra(ethylhe xanoate) | 15 g ascorbyl tetrapalmi tate | 5:1 | 4.3:1 | 160 | 1.4:1 |
| Example 4 | 1 | 20 | 5% | 75 g pentaerythrit yl tetra(ethylhe xanoate) | 15 g retinyl propionat e | 5:1 | 4.3:1 | 111 | 1:1 |
| Example 5 | 1 | 20 | 5% | 75 g pentaerythrit yl tetra(ethylhe xanoate) | 15 g retinyl propionat e | 5:1 | 4.3:1 | 444 | 4:1 |
| Example 6 | 1 | 20 | 5% | 80 g pentaerythrit yl tetra(ethylhe xanoate) | 10 g retinyl propionat e | 8:1 | 4.3:1 | 160 | 1.4:1 |
| Example 7 | 0.45 | 8.55 | 5.3% | 75 g pentaerythrit yl tetra(ethylhe xanoate) | 15 g retinyl propionat e | 5:1 | 10:1 | 160 | 1.6:1 |
| Example 8 | 1.5 | 28.5 | 5.3% | 75 g pentaerythrit yl tetra(ethylhe xanoate) | 15 g retinyl propionat e | 5:1 | 3:1 | 200 | 1.7:1 |
| Example 9 | 0.75 | 6.75 | 11.1% | 20.5 g pentaerythrit yl tetra(ethylhe xanoate) | 2 g phenyleth yl resorcinol | 10.3:1 | 3:1 | 70 | 2.3:1 |
| Comparat ive Example 1 | 1 | 20 | 5% | 75 g oleic acid | 15 g retinyl propionat e | 5:1 | 4.3:1 | 160 | 1.4:1 |
| Comparat ive Example 2 | 1 | 20 | 5% | 75 g GTCC | 15 g retinyl propionat e | 5:1 | 4.3:1 | 160 | 1.4:1 |
| Comparat ive Example 3 | 1 | 20 butylene glycol | 5% | 75 g pentaerythrit yl tetra (ethylhexano ate) | 15 g retinyl propionat e | 5:1 | 4.3:1 | 160 | 1.4:1 |
| Comparat ive Example 4 | 1 | 20 | 5% | 75 g GTCC | 15 g bakuchiol | 5:1 | 4.3:1 | 160 | 1.4:1 |
| Comparat ive Example 5 | 1 | 20 | 5% | 75 g pentaerythrit yl tetra (ethylhexano ate) | 15 g retinyl propionat e | 5:1 | 4.3:1 | 100 | 0.9:1 |
| Comparat ive Example 6 | 0.5 | 20.5 | 2.4% | 75 g pentaerythrit yl tetra(ethylhe xanoate) | 15 g retinyl propionat e | 5:1 | 4.3:1 | 160 | 1.4:1 |
| Comparat ive Example 7 | 0.4 | 8 | 5% | 75 g pentaerythrit yl tetra(ethylhe xanoate) | 15 g retinyl propionat e | 5:1 | 10.7:1 | 138 | 1.4:1 |
| Comparat ive Example 8 | 1 | 20 | 5% | 60 g pentaerythrit yl tetra(ethylhe xanoate) | 30 g retinyl propionat e | 2:1 | 4.3:1 | 160 | 1.4:1 |
| Comparat ive Example 9 | 0.4 | 7.1 | 5.6% | 28 g pentaerythrit yl tetra(ethylhe xanoate) | 2 g phenyleth yl resorcinol | 14:1 | 4:1 | 62.5 | 1.67:1 |

**Table 2: Results of Active Substance Degradation Rate Determination**

| | Retention rate at 0 d (%) | Retention rate at 7 d (%) | Retention rate at 15 d (%) | Retention rate at 28 d (%) |
|---|---|---|---|---|
| Example 1 | 100 | 96.3 | 94.5 | 90.2 |
| Example 2 | 100 | 96.9 | 91.2 | 82.9 |
| Example 3 | 100 | 83.6 | 76.3 | 68.8 |
| Example 4 | 100 | 96.0 | 94.3 | 90.1 |
| Example 5 | 100 | 96.2 | 94.5 | 90.2 |
| Example 6 | 100 | 96.1 | 94.4 | 90.3 |
| Example 7 | 100 | 95.9 | 94.2 | 90.1 |
| Example 8 | 100 | 96.5 | 94.1 | 90.2 |
| Example 9 | 100 | 96.3 | 93.8 | 90.3 |
| Comparative Example 2 | 100 | 94.5 | 85.0 | precipitate |
| Comparative Example 4 | 100 | 89.6 | 73.2 | precipitate |
| Comparative Example 8 | 100 | 76.5 | 62.5 | 35.2 |

It can be seen from the results in Table 2 that the emulsions of the Examples of the present invention had a higher retention rate over time compared with those of Comparative Examples 2 and 4. In contrast to GTCC, pentaerythrityl tetra(ethylhexanoate), the oil-phase component in the solution of the present invention, exhibited better compatibility effects with other components and could maintain a higher retention rate even after 28 days, whereas the emulsions of Comparative Examples 2 and 4 suffered from precipitation after 28 days.

In addition, based on the experimental results recorded in Examples 1 to 9 and Comparative Examples 1 to 9 as well as the experimental results shown in Figs. 2 to 9, it can be concluded that when the components of the emulsion in the solution of the present invention are compounded in appropriate proportions, emulsion products can be successfully prepared and obtained while achieving an excellent protective effect on the oil-phase active substance. On the contrary, the use of other components for compounding or inappropriate compounding proportions leads to problems such as easy precipitation, failure to form an emulsion, inability to form an oil gel, and severe discoloration. As can be seen from Fig. 5, the emulsion of Comparative Example 4 exhibited demulsification after storage, with a large amount of bakuchiol precipitated and severe discoloration observed. Fig. 6 shows that Comparative Example 3 presented severe discoloration after storage, while in Comparative Example 1, the oil, lipopeptide and glycerol failed to form an oil gel upon mixing and instead exhibited a delamination phenomenon. It can be seen from Fig. 7 that Comparative Example 7 had a poor-quality oil gel with the presence of free oil. Fig. 9 shows that the emulsion of Comparative Example 9 showed obvious delamination, indicating poor emulsion stability.

In the solutions of the Examples of the present invention, by introducing sodium surfactin and optimally selecting glycerol and pentaerythrityl tetra(ethylhexanoate) to be compounded with oil-phase active substance as well as their optimal compounding proportions, the solutions in the examples achieve an excellent protective effect on oil-phase active substance. A retention rate of more than 90% for oil-phase active substance can be maintained after 28 days, which effectively reduces the degradation of oil-phase active substance.

The above description is merely illustrative of the examples of the present invention, and the protection scope of the present invention is not limited by these specific examples but is defined by the claims of the present invention. Various modifications and variations of the present invention may be made by those skilled in the art. Any modification, equivalent substitution, improvement or the like made within the technical concept and principles of the present invention shall fall within the protection scope of the present invention.

## Claims

1. An oil gel, comprising:
(a) at least one lipopeptide;
(b) glycerol;
(c) pentaerythrityl tetra(ethylhexanoate); and
(d) at least one oil-phase active substance.

2. The oil gel according to claim 1, wherein the weight percentage of the at least one lipopeptide relative to glycerol is not less than 3%.

3. The oil gel according to claim 1, wherein the weight ratio of the at least one oil-phase active substance to pentaerythrityl tetra(ethylhexanoate) is 1:(3 to 13).

4. The oil gel according to claim 1, wherein the ratio of the total weight of the at least one lipopeptide and glycerol to the total weight of the at least one oil-phase active substance and pentaerythrityl tetra(ethylhexanoate) is 1:(3 to 10).

5. The oil gel according to claim 2, wherein the weight percentage of the at least one lipopeptide relative to glycerol is not less than 5%.

6. The oil gel according to claim 5, wherein the weight percentage of the at least one lipopeptide relative to glycerol is 5% to 12%.

7. The oil gel according to claim 3, wherein the weight ratio of the at least one oil-phase active substance to the pentaerythrityl tetra(ethylhexanoate) is 1:(5 to 11).

8. The oil gel according to claim 1, wherein the at least one lipopeptide comprises any one or more selected from sodium surfactin, iturin, fengycin, daptomycin, polymyxin, bacillomycin L, plipastatin and bacillomycin D.

9. The oil gel according to claim 1, wherein the at least one oil-phase active substance comprises one or more selected from retinol, retinyl acetate, retinyl propionate, retinyl palmitate, bakuchiol, equol, nervonic acid, ascorbyl palmitate, ascorbyl tetrapalmitate and phenylethyl resorcinol.

10. An emulsion, comprising water and the oil gel according to any one of claims 1 to 9.

11. The emulsion according to claim 10, wherein the ratio of the total weight of the at least one lipopeptide, glycerol, at least one oil-phase active substance and pentaerythrityl tetra(ethylhexanoate) to the weight of water is 1:(1 to 4).

12. A preparation method of the oil gel according to any one of claims 1 to 9, wherein the method comprises the following steps:
1) dissolving the at least one lipopeptide in glycerol, and heating and stirring the mixture to obtain a solution A;
2) dissolving the at least one oil-phase active substance in pentaerythrityl tetra(ethylhexanoate), and stirring the mixture to obtain a solution B; and
3) adding the solution A to the solution B under the conditions of constant temperature water bath and stirring, to obtain the oil gel.

13. A preparation method of the emulsion according to claim 11 or 12, wherein the method further comprises adding an aqueous phase to the oil gel under stirring conditions and mixing them thoroughly to obtain the lipopeptide-containing emulsion.

14. Use of the oil gel according to any one of claims 1 to 9 or the emulsion according to claim 10 or 11 in cosmetics.
